Europäisches Patentamt

European Patent Office

Office européen des brevets

(19)

(11) Numéro de publication: **0 381 577**
**A2**

(12) **DEMANDE DE BREVET EUROPEEN**

(21) Numéro de dépôt: **90400253.2**

(22) Date de dépôt: **30.01.90**

(51) Int. Cl.5: **A61M 5/32**

(30) Priorité: **03.02.89 FR 8901387**

(43) Date de publication de la demande:
**08.08.90 Bulletin 90/32**

(84) Etats contractants désignés:
**AT BE CH DE DK ES GB GR IT LI LU NL SE**

(71) Demandeur: **Boumendil, Frank**
**41, Avenue Victor-Hugo**
**F-92100 Boulogne(FR)**

Demandeur: **Gordon, Michel**
**7, Avenue Fréniet**
**F-75016 Paris(FR)**

(72) Inventeur: **Boumendil, Frank**
**41, Avenue Victor-Hugo**
**F-92100 Boulogne(FR)**

(74) Mandataire: **Tony-Durand, Serge**
**Cabinet Tony-Durand 77, rue Boissière**
**F-75116 Paris(FR)**

(54) **Capuchon de protection pour l'aiguille d'une seringue d'injection.**

(57) Ce capuchon de protection est constitué par un corps creux (1) de forme allongée, ouvert à un bout et qui présente, sur toute sa longueur, une ouverture (9) d'introduction de l'aiguille (2) d'une seringue (3). Du côté opposé, ce capuchon comporte une face plate (8) d'appui permettant de le poser de façon stable sur une surface place. De plus il est prévu des lamelles flexibles (11) susceptibles de servir d'organes de blocage du manchon (4) de montage de l'aiguille (2) contre la paroi interne du capuchon, après son introduction.

Ce capuchon de protection est destiné à enfermer l'aiguille d'une seringue d'injection avant et après emploi de celle-ci.

FIG. 3

EP 0 381 577 A2

## Capuchon de protection pour l'aiguille d'une seringue d'injection

La présente invention concerne les capuchons de protection destinés à enfermer l'aiguille d'une seringue d'injection à usage médical ou non.

Actuellement ces capuchons affectent la forme d'un corps creux allongé de section circulaire dont une extrémité est fermée cependant que l'autre est ouverte de façon à pouvoir être emboitée avec frottement sur le manchon conique, prévu à la base de l'aiguille à recouvrir, pour sa fixation sur l'embout de sortie d'une seringue. Ces capuchons peuvent ainsi être aisément retirés lors de l'utilisation des aiguilles correspondantes. Cependant ils sont destinés à être remis en place après utilisation de ces aiguilles de façon à éviter tout risque de blessure d'un tiers et surtout tout danger de contamination dû au sang de la personne ayant subi une injection, notamment tout danger de contamination par le Sida ou l'Hépatite B, etc.

Toutefois, la ré-introduction d'une telle aiguille à l'intérieur d'un capuchon de ce genre est très malaisée. En effet, l'opérateur doit tenir ce capuchon avec une main tout en tenant, avec l'autre, la seringue portant l'aiguille usagée. Il doit alors engager l'extrémité de cette aiguille à l'intérieur du capuchon, ou vice-versa. Mais cette manoeuvre est très délicate, et très fréquemment l'opérateur ne parvient pas à engager l'aiguille à l'intérieur de son capuchon. Or, une mauvaise manipulation peut se traduire par une piqure intempestive de l'opérateur, donc un risque de contamination de celui-ci. Dans ces conditions le personnel médical est de plus en plus enclin à ne plus remettre en place les capuchons de protection sur les aiguilles d'injection, ce qui entraîne des risques de contamination de tierces personnes.

Pour tenter de résoudre ce problème, le brevet US 4 643 722 a pour objet un capuchon de protection comportant une fente qui s'étend à peu près sur toute sa longueur à partir de son extrémité ouverte. Cette fente est prévue pour permettre l'introduction d'une aiguille par le côté et non plus en bout. Toutefois ceci ne suffit pas pour résoudre le problème en cause. En effet, pour introduire une aiguille de seringue à l'intérieur d'un tel capuchon, l'opérateur doit encore tenir celui-ci avec une main alors qu'avec l'autre il tient la seringue portant l'aiguille usagée. Il existe donc encore des risques importants de piqure intempestive du seul fait que l'opérateur doit tenir le capuchon avec une main pendant l'introduction d'une aiguille à l'intérieur de celui-ci.

Un autre problème non résolu de façon satisfaisante par le brevet US 4 643 722 est celui de l'enfermement complet d'une aiguille usagée à l'intérieur de son capuchon de protection. En effet, pour supprimer tout risque de contamination, il faut qu'une telle aiguille soit parfaitement immobilisée et complètement enfermée dans son capuchon de protection. Or, pour fermer la fente longitudinale le brevet US 4 643 722 prévoit simplement de fermer la fente longitudinale d'introduction de l'aiguille avec un organe rapporté ensuite sur cette fente. La mise en place de cet organe de fermeture est encore une cause de piqure accidentelle de l'opérateur si l'aiguille vient à bouger pendant cette opération. Par ailleurs, l'adjonction d'un tel organe de fermeture est une cause de complication, non seulement en ce qui concerne les opérations à réaliser, mais également pour la fabrication du capuchon de protection et son prix de revient, qui doit rester extrêmement faible.

C'est pourquoi la présente invention a pour objet un capuchon de protection destiné au même usage mais qui est conçu de façon à éviter les inconvénients rappelés ci-dessus et surtout les risques de contamination d'un praticien ou d'une tierce personne.

A cet effet l'invention a pour objet un capuchon de protection pour l'aiguille d'une seringue d'injection comportant, à sa base, un manchon de montage, ce capuchon étant constitué par un corps creux de forme allongée, ouvert à un bout, et présentant sur toute sa longueur une ouverture longitudinale pour l'introduction latérale de l'aiguille d'une seringue, à travers le côté correspondant de ce corps creux, caractérisé en ce que :

- du côté opposé à cette ouverture longitudinale, ce corps allongé comporte une face plate d'appui permettant de le poser de façon stable sur une surface plate,

- et en ce que, de part et d'autre de l'ouverture longitudinale, il est prévu des lamelles flexibles aptes à laisser passer l'aiguille usagée d'une seringue lors de son introduction à travers l'ouverture et à fermer ensuite l'espace intérieur du capuchon de protection pour y enfermer l'aiguille usagée placée dans cet espace.

Ainsi, la ré-introduction d'une aiguille d'injection à l'intérieur d'un tel capuchon est extrêmement facile à réaliser. En effet, pour cette opération ce capuchon peut être posé sur une surface plane et il suffit alors d'introduire l'aiguille par le dessus de ce capuchon en l'engageant dans l'ouverture qui s'étend sur toute la longueur de celui-ci. Lors de l'engagement de l'aiguille à l'intérieur du capuchon, le manchon de montage existant à sa base, écarte momentanément les lamelles de blocage qui se rapprochent ensuite pour enfermer l'aiguille usagée placée à l'intérieur du capuchon.

Cependant le principal avantage du capuchon selon l'invention réside dans le fait que celui-ci ne doit pas être tenu à la main lors de l'introduction d'une aiguille usagée, puisqu'il peut alors être posé à plat sur un support quelconque. Ceci supprime donc radicalement tout risque de piqûre accidentelle de l'opérateur.

Dans une forme de réalisation avantageuse les lamelles flexibles prévues de part et d'autre de l'ouverture longitudinale du capuchon sont inclinées vers l'intérieur et elles sont aptes à faire pression sur le manchon de montage existant à la base d'une aiguille placée à l'intérieur de ce capuchon, afin d'appliquer ce manchon contre la paroi intérieure dudit capuchon.

En plus de ces lamelles de blocage inclinées vers l'intérieur, le capuchon de protection selon l'invention peut avantageusement comporter deux autres lamelles flexibles recouvrant celles-ci et formant un clapet de fermeture au dessus de celles-ci.

Cependant d'autres particularités et avantages du capuchon de protection selon l'invention apparaitront au cours de la description suivante d'un exemple de celui-ci. Cette description est donnée en référence aux dessins annexés à simple titre indicatif, et sur lesquels :

La figure 1 est une vue en perspective d'une seringue d'injection dont l'aiguille est enfermée dans un capuchon de protection selon l'invention.

La figure 2 est une vue en coupe axiale de ce capuchon de protection.

Les figures 3 et 4 en sont des vues en coupe transversale selon les lignes III-III et IV-IV de la figure 2.

La figure 5 est une vue en élévation de l'extrémité du présent capuchon qui est accolée à une seringue lors de sa mise en place.

La figure 6 est une vue en perspective illustrant l'opération de remise en place d'une aiguille d'injection à l'intérieur d'un tel capuchon de protection.

La figure 7 est une vue schématique en coupe similaire à la figure 3 et qui illustre cette même opération.

Ainsi qu'il a déjà été indiqué, le capuchon de protection 1 selon l'invention est destiné à recouvrir et à enfermer une aiguille 2 équipant une seringue d'injection 3. De la façon habituelle, cette aiguille est pourvue à sa base d'un manchon conique 4 destiné à permettre le montage de cette aiguille sur une telle seringue par adaptation de ce manchon sur l'embout de sortie 5 de la seringue correspondante, ce manchon 4 comportant une collerette 6.

Le capuchon 1 est constitué par un corps creux de forme allongée qui est fermé à l'une de ses extrémités 7, cependant que son extrémité opposée est librement ouverte. Dans l'exemple représenté, ce corps creux présente une section de forme trapézoïdale dont la grande base correspond à une face plate 8 susceptible de servir de surface d'appui sur un support plan quelconque. Sur sa face opposée, qui correspond donc à la petite base de sa section trapézoïdale, le capuchon 1 présente une fente 9 s'étendant sur toute sa longueur depuis son extrémité ouverte. De part et d'autre de cette fente, il est prévu deux lamelles flexibles 10 qui forment en quelque sorte un clapet de fermeture. La flexibilité élastique de ces lamelles est obtenue grâce à la fabrication du capuchon 1 par moulage en une matière plastique appropriée.

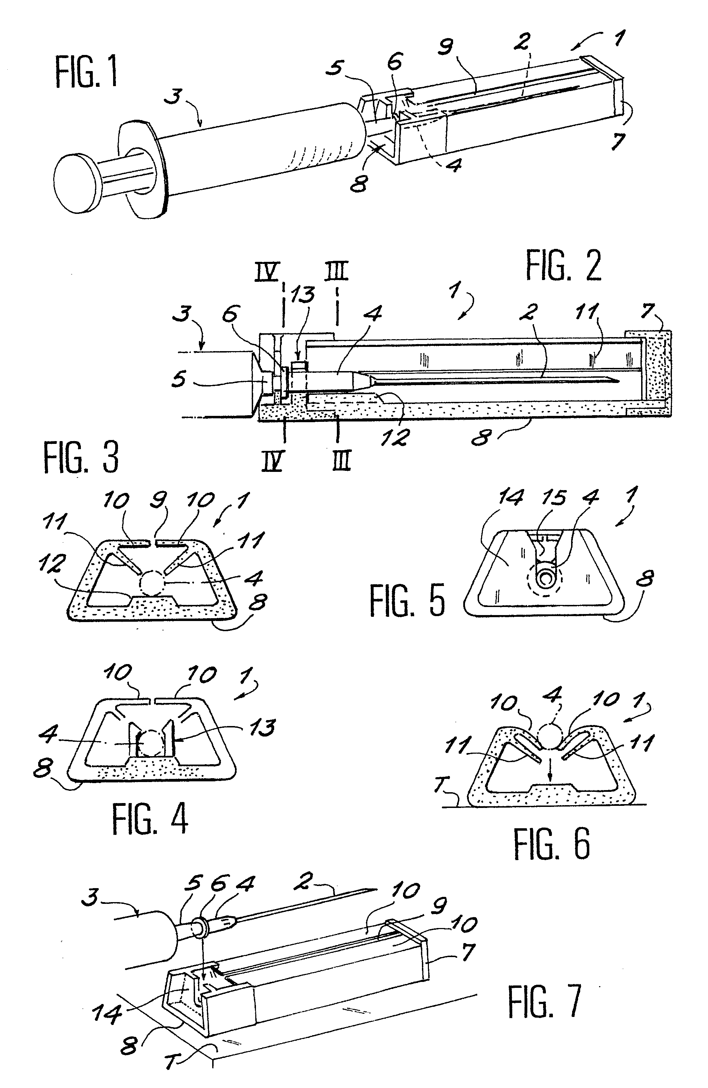

Au-dessous des deux lamelles 10, il est prévu deux autres lamelles flexibles 11 qui sont inclinées vers l'intérieur. Ces lamelles ont pour fonction de servir d'organes de blocage pour le manchon 4 de montage de l'aiguille 2, lorsque celle-ci est en place à l'intérieur du capuchon. Ce manchon peut alors reposer sur une plaquette d'appui 12 venue de moulage sur la face interne de la grande face 8 du capuchon. Comme on peut le constater d'après les figures 2 et 3, le capuchon 1 est ainsi parfaitement bloqué sur l'aiguille 2 et son manchon 4, du fait même de la pression élastique exercée par les deux lamelles 11 sur ce manchon.

Cependant la fixation en place du capuchon 1 peut encore être renforcée en prévoyant une pince élastique 13 à l'entrée de ce capuchon, cette pince étant agencée pour s'adapter élastiquement sur le manchon 4 de montage de l'aiguille (voir figure 4). Toutefois cette pince élastique n'est pas absolument indispensable pour la fixation en place du capuchon 1. Dans la forme de réalisation représentée, une cloison 14 présentant une encoche 15 est prévue à l'extrémité du capuchon qui est opposée à son extrémité fermée 7. Mais là encore cette cloison n'est pas indispensable.

Le retrait du présent capuchon peut être réalisé très facilement de la même façon que pour un capuchon classique, c'est-à-dire par dégagement de l'aiguille 2 hors de celui-ci par translation dans le sens axial. Cependant grâce à la conception particulière du présent capuchon, celui-ci peut être ensuite remis en place très facilement après utilisation de l'aiguille, et ce sans aucun risque de piqûre accidentelle, et par suite de contamination.

Grâce à la grande face plate 8, ce capuchon peut alors être posé sur une surface plate, par exemple une table T, comme représenté sur la figure 5. Il suffit ensuite de présenter l'aiguille 2 au dessus de la fente 9 du capuchon pour l'introduire par dessus à l'intérieur de ce dernier. Lors de cette introduction, l'aiguille 2 écarte momentanément les deux lamelles 10 formant clapet de fermeture. Puis le manchon 4 existant à la base de cette aiguille écarte à son tour les deux lamelles intérieures 11 de blocage jusqu'à ce que ce manchon vienne reposer contre la plaque d'appui 12. Les lamelles 11 reviennent alors dans leur position initiale de sorte qu'elles font pression, par leurs bords, contre le manchon 4, ce qui assure l'immobilisation en place du capuchon 11 sur l'aiguille 2.

Ainsi, il est inutile à l'opérateur d'utiliser ses deux mains pour l'opération de remise en place du capuchon 1, puisque celui-ci peut alors être posé sur une table T ou tout autre support plan voulu. Par ailleurs, il n'y a aucun risque de mauvaise manipulation car il suffit de présenter l'aiguille 2 au dessus de la face 9 et de l'enfoncer ensuite entre les deux séries de lamelles 10 et 11 jusque dans sa pôsition définitive. En conséquence, le praticien ne risque en aucune façon de se blesser à cette occasion, car il tient alors d'une seule main la seringue 3 sans avoir à tenir le capuchon 1 avec l'autre main, comme cela doit être le cas avec les capuchons actuels de protection.

Un autre avantage du présent capuchon réside dans le fait que l'aiguille 2 placée à l'intérieur de ce capuchon s'y trouve parfaitement immobilisée et enfermée par les deux paires de lamelles 10 et 11 d'immobilisation et de fermeture, sans qu'il soit nécessaire de rajouter un organe quelconque de fermeture. Or ceci est également indispensable pour éviter tout risque de piqûre accidentelle et de contamination.

Il convient de noter que le capuchon selon l'invention n'est pas limité au seul exemple de réalisation décrit ci-dessus. Ainsi, ce capuchon pourrait présenter une section autre qu'une section de forme trapézoïdale. Eventuellement cette section pourrait être de forme rectangulaire ou carrée. A la limite, ce capuchon pourrait même comporter une section circulaire ou elliptique comportant un méplat constituant sa grande face 8 d'appui. Comme déjà mentionné, la pince élastique 13 pourrait être éventuellement supprimée. Cependant la position de cette pince pourrait également être modifiée car celle-ci pourrait être disposée de façon à se trouver placée de l'autre côté par rapport à la collerette 6 prévue à la base du cône 4 servant de support à l'aiguille. Du reste, de nombreuses autres variantes peuvent être envisagées pour le présent capuchon de protection.

## Revendications

1. Capuchon de protection pour l'aiguille d'une seringue d'injection comportant à sa base un manchon de montage, ce capuchon étant constitué par un corps creux de forme allongée , ouvert à un bout et présentant sur toute sa longueur, une ouverture longitudinale (9) pour l'introduction latérale de l'aiguille (2) d'une seringue (3) à travers le côté correspondant de ce corps creux, caractérisé en ce que :
- du côté opposé à cette ouverture longitudinale (9), ce corps allongé (1) comporte une face plate (8) d'appui permettant de le poser de façon stable sur une surface plate,
et en ce que, de part et d'autre de l'ouverture longitudinale (9), il est prévu des lamelles flexibles (11) aptes à laisser passer l'aiguille usagée (2) d'une seringue lors de son introduction à travers l'ouverture (9) et à fermer ensuite l'espace intérieur du capuchon de protection pour y enfermer l'aiguille usagée placée dans cet espace.

2. Capuchon de protection selon la revendication 1, caractérisé en ce que les lamelles flexibles (11), prévues de part et d'autre de l'ouverture longitudinale (9) de ce capuchon, sont inclinées vers l'intérieur, et elles sont aptes à faire pression sur le manchon (4) de montage existant à la base d'une aiguille (2).

3. Capuchon de protection selon la revendication 2, caractérisé en ce qu'en plus des lamelles de blocage (11), qui sont inclinées vers l'intérieur, il est prévu deux autres lamelles flexibles (10) recouvrant celles-ci et formant un clapet de fermeture de l'ouverture (9) d'introduction.

4. Capuchon de protection selon l'une des revendications précédentes, caractérisé en ce qu'il présente une section de contour quadrangulaire, et de préférence une section de forme trapézoïdale dont la grande base correspond à la face plate (8) d'appui de ce capuchon, qui est situé du côté opposé à l'ouverture longitudinale (9).

5. Capuchon de protection selon l'une des revendications précédentes, caractérisé en ce que près de l'extrémité ouverte de celui-ci, il est prévu une pince élastique (13) de réception et de blocage du manchon (4) existant à la base d'une aiguille (2) de seringue d'injection.

6. Capuchon de protection selon l'une des revendications précédentes, caractérisé en ce que près de l'extrémité ouverte de celui-ci, il est prévu une cloison (14) présentant une encoche (15).

FIG. 1

FIG. 2

FIG. 3

FIG. 4

FIG. 5

FIG. 6

FIG. 7